# EUROPEAN PATENT SPECIFICATION

(11) **EP 4 328 320 B1**
(45) Date of publication and mention of the grant of the patent: **29.04.2026**
(21) Application number: 22791731.7
(22) Date of filing: 19.04.2022
(51) Int. Cl.: C12Q 1/26, C12Q 1/44, G01N 33/53, G01N 33/92

(54) **REAGENT COMPOSITION AND KIT**
REAGENZZUSAMMENSETZUNG UND KIT
COMPOSITION DE RÉACTIF ET KIT

(30) Priority: 19.04.2021 JP 2021070615
(43) Date of publication of application: 28.02.2024
(73) Proprietor: Denka Company Limited, Tokyo 103-8338 (JP)
(72) Inventor: ITO, Yasuki, Tokyo 103-8338 (JP); ABE, Junichi, Tokyo 103-8338 (JP); OKADA, Hiromasa, Tokyo 103-8338 (JP)
(74) Representative: Schnappauf, Georg
(86) International application number: PCT/JP2022/018185
(87) International publication number: WO 2022/224960

(56) References cited:
- EP-A1- 3 779 456
- WO-A1-2016/017547
- WO-A1-2020/032079
- WO-A1-2023/199890
- JP-A- 2005 162 780
- JP-A- 2007 297 519
- JP-A- 2007 325 587
- JP-A- 2009 149 743
- JP-A- 2017 209 035
- JP-A- 2021 040 516
- JP-A- 2021 040 520
- US-A1- 2010 035 288
- ITO YASUKI ET AL: "Development of a homogeneous assay for measurement of small dense LDL cholesterol", CLINICAL CHEMISTRY, OXFORD UNIVERSITY PRESS, US, vol. 57, no. 1, 1 January 2011 (2011-01-01), pages 57 - 65, XP009144502, ISSN: 0009-9147, DOI: 10.1373/CLINCHEM.2010.149559

## Description

### TECHNICAL FIELD

The present invention relates to a reagent composition and a kit.

### BACKGROUND ART

As a technique relating to a method of measuring LDL cholesterol, there is a method described in Patent Document 1 (Japanese Unexamined Patent Publication No. 2000-325097). The document discloses a method of measuring lipoprotein cholesterol including a first step of selectively subjecting HDL cholesterol to an enzyme reaction by adding an enzyme and a first surfactant to a sample containing lipoproteins, subsequently, a second step of selectively subjecting LDL cholesterol to an enzyme reaction by adding a second surfactant, and measuring HDL cholesterol and/or LDL cholesterol by measuring a compound consumed or produced by the reaction with the enzyme in the first or second step (Claim 1). Then, according to such a method, LDL cholesterol can be simply and inexpensively quantified without requiring a lipoprotein aggregating agent that increases the turbidity of the reaction solution, without limitation on the enzymes to be used, and without the need to add another new enzyme in the step of reacting LDL cholesterol. Further, if necessary, a measurement method and a measurement reagent capable of accurately and inexpensively measuring HDL cholesterol without forming lipoprotein aggregates that interfere with optical measurement can be provided. Therefore, it is considered that the method is particularly useful in the field of clinical tests for atherosclerosis and the like (Paragraph 0055). Further prior art is disclosed in document US2010/035288.

### RELATED DOCUMENT

### PATENT DOCUMENT

[Patent Document 1] Japanese Unexamined Patent Publication No. 2000-325097

### SUMMARY OF THE INVENTION

### TECHNICAL PROBLEM

The present inventors have investigated the measurement of small dense LDL cholesterol (sdLDL-C) among LDL cholesterols. Then, it has been found that there is room for improvement in terms of measurement accuracy in the technique described in Patent Document 1.

The present invention provides a technique for measuring sdLDL-C with excellent accuracy.

### SOLUTION TO PROBLEM

According to the present invention, the following reagent composition and kit are provided.
[1] A reagent composition that is used as a first reagent composition for a method of quantifying small dense LDL cholesterol (sdLDL-C) in a sample, the method including:
   causing the first reagent composition to act on the sample; and
   after the causing the first reagent composition to act on the sample, applying a second reagent composition for quantifying the sdLDL-C to quantify cholesterol in a remaining lipoprotein,
   in which the first reagent composition has one or two or more activities selected from the group consisting of cholesterol esterase activity, cholesterol oxidase activity, and sphingomyelinase activity, and contains polyoxyethylene monostyrenated phenyl ether.
[2] The reagent composition according to [1], in which a degree of polymerization n of polyoxyethylene in the polyoxyethylene monostyrenated phenyl ether is 5 or more and 80 or less.
[3] The reagent composition according to [1] or [2], in which a content of the polyoxyethylene monostyrenated phenyl ether in the reagent composition is 0.05% (w/v) or more and 0.6% (w/v) or less with respect to the entire reagent composition.
[4] The reagent composition according to any one of [1] to [3], in which the first reagent composition further comprises at least one activity selected from the group consisting of peroxidase activity and catalase activity.
[5] The reagent composition according to any one of [1] to [4], in which the first reagent composition comprises any one of a hydrogen donor and a coupler.
[6] A kit used for quantifying the sdLDL-C in the sample, including:
   a first reagent composition according to any one of [1] to [5]; and
   a second reagent composition for quantifying the sdLDL-C.
[7] The kit according to [6], in which the second reagent composition has peroxidase activity.
[8] The kit according to [6] or [7], in which
   (i) the first reagent composition comprises a hydrogen donor and does not comprise a coupler, and the second reagent composition does not comprise the hydrogen donor and comprises the coupler;
      or
   (ii) the first reagent composition does not comprise a hydrogen donor and comprises a coupler, and the second reagent composition comprises the hydrogen donor and does not comprise the coupler.

### ADVANTAGEOUS EFFECTS OF INVENTION

According to the present invention, it is possible to provide a technique for measuring sdLDL-C with excellent accuracy.

### BRIEF DESCRIPTION OF THE DRAWINGS

of measured values of sdLDL-C.
[Fig. 2] A diagram showing evaluation results of accuracy of measured values of sdLDL-C.
[Fig. 3] A diagram showing evaluation results of accuracy of measured values of sdLDL-C.

### DESCRIPTION OF EMBODIMENTS

Hereinafter, embodiments will be described. In the present embodiment, the composition such as the measurement reagent may contain each component alone or in combination of two or more. In addition, in the present specification, a numerical range "x to y" indicates "x or more and y or less", and includes both a lower limit value x and an upper limit value y.

First, a lipoprotein will be described. A lipoprotein is fractionated roughly into Very Low Density Lipoprotein (VLDL), Low Density Lipoprotein (LDL) and High Density Lipoprotein (HDL), and LDL is sub-fractionated into a small dense LDL (sdLDL), and other sub-fractions. sdLDL is also referred to as small particle LDL, small LDL (SLDL), dense LDL, or small and dense LDL, and other LDLs are sometimes referred to as large LDL (L LDL), light LDL, or large buoyant LDL (lbLDL).

These lipoprotein fractions and sub-fractions may be distinguished based on particle size or density. The particle size of lipoprotein in diameter is, 30 nm to 80 nm (or 30 nm to 75 nm) for VLDL, 22 nm to 28 nm (or 19 nm to 30 nm) for LDL, and 7 nm to 10 nm for HDL, although the figures may vary depending on the researchers.

The density of lipoprotein is, for example, 1.006 g/cm³ or less for VLDL, 1.019 to 1.063 g/cm³ for LDL, and 1.063 to 1.21 g/cm³ for HDL.

Among lipoproteins, the diameter of LDL particles can be measured by, for example, gradient gel electrophoresis (GGE) (JAMA, 260, p. 1917-21, 1988) or NMR (HANDBOOK OF LIPOPROTEIN TESTING 2nd Edition, Edited by Nader Rifai et al. p. 609-623, AACC PRESS: The Fats of Life Summer 2002, LVDD 15 YEAR ANNIVERSARY ISSUE, Volume AVI No. 3, p. 15-16). The density can be determined based on, for example, analysis by ultracentrifugation (Atherosclerosis, 106, p.241-253, 1994: Atherosclerosis, 83, p. 59, 1990).

In the present embodiment, the sdLDL to be measured generally refers to a sub-fraction having a diameter of about 22.0 to about 25.5 nm or a sub-fraction having a density of 1.040 to 1.063 g/cm³ among LDL fractions.

The reason why LDL is sub-fractionated according to the particle size is that a small LDL among LDLs needed to be fractionally measured since LDL with a small particle diameter causes more arteriosclerosis and is higher in malignancy than other LDLs. Since the distributions of diameter and density of a LDL are continuous, it is not possible to determine the value of density above which the malignancy is clearly higher. Therefore, the density value of 1.040 to 1.063 g/cm³ described above is not an established characteristic of sdLDL, but is a value on the high density side obtained by dividing the density range of 1.019 to 1.063 g/cm³, which is widely used and established as the density of LDL, at a median point. For example, regarding the density of sdLDL, in a different report, sdLDL is fractionated in a range of 1.044 to 1.060 g/cm³ (Atherosclerosis: 106 241-253 1994). There are some differences among researchers on how to set the range of the density of sdLDL or the like, but with any of the ranges chosen, the presence of sdLDL is associated with clinical malignancy.

In the present specification, specifically, a sdLDL is defined as an LDL having a high density among LDLs and with a higher association with arteriosclerosis clinically than other LDLs. In addition, preferably, the sdLDL has a higher density than the median point within the range of density for LDLs, and more preferably, the sdLDL refers to an LDL with the density in a range of 1.044 to 1.063 g/cm³. Further, lipoproteins other than LDL refer to VLDL or HDL, and may include chylomicrons, intermediate density lipoproteins (IDL), or very high density lipoproteins (VHDL).

The present inventor has conducted investigations to improve the accuracy when quantifying sdLDL-C in a sample. As a result, it has been found that when sdLDL cholesterol (sdLDL-C) is quantified by a method including causing a first reagent composition to act on a sample (first step), and then applying a second reagent composition for quantifying the sdLDL-C to quantify cholesterol in a remaining lipoprotein (second step), in the first step, it is important to highly control the selectivity of the action of the first reagent composition on LDLs other than sdLDL. More specifically, in the first step, it is important that the first reagent composition selectively acts on LDLs other than sdLDL, and when the selectivity is too low, that is, the first reagent composition is a reagent that easily acts on sdLDL as a measurement target, there is a concern that the accuracy of the quantification of sdLDL-C in the sample in the second step may be decreased.

Therefore, as a result of further investigations conducted by the present inventors to make the selectivity of the action of the first reagent composition more preferable, it has been found that the first reagent composition is configured to have a specific enzyme activity and contain a specific surfactant, the first reagent composition that acts on LDLs other than sdLDL with high selectivity can be stably obtained.

Further, it is known that a polyoxyethylene styrenated phenyl ether derivative or polyoxyethylene distyrenated phenyl ether may be used as a surfactant. However, during intensive studies, the present inventors have newly found that, among polyoxyethylene styrenated phenyl derivatives, the presence of polyoxyethylene monostyrenated phenyl ether increases the selectivity to L LDL when the sample reacts with the first reagent composition, and as a result, sdLDL-C can be accurately measured.

Hereinafter, each reagent composition will be described in more detail.

### (Reagent Composition (First Reagent Composition))

In this embodiment, the reagent composition is used as the first reagent composition of a method for quantifying sdLDL-C in a sample, which includes the steps of acting a first reagent composition on the sample and then acting a second reagent composition for quantifying sdLDL-C to quantify cholesterol in the remaining lipoproteins. The property of the first reagent composition is specifically liquid.

Hereinafter, the reagent composition used as the first reagent composition is also simply referred to as a "first reagent composition".

The first reagent composition has one or two or more activities selected from the group consisting of cholesterol esterase activity, cholesterol oxidase activity, and sphingomyelinase activity and contains polyoxyethylene monostyrenated phenyl ether.

In the present embodiment, since the first reagent composition contains a specific nonionic surfactant and has a specific enzyme activity, when the first reagent composition is added to the sample, it can stably act on and eliminate cholesterol in LDLs other than sdLDL in the sample with high selectivity. In addition, cholesterol in LDLs other than sdLDL can be stably led to the outside of the reaction system with high selectivity.

Here, the expression "surfactant acts (reacts)" refers to that a surfactant degrades lipoproteins to liberate cholesterol in the lipoproteins. For example, in the case of "surfactant that acts on (reacts with) lipoproteins other than sdLDL", it is not required that the surfactant does not act on sdLDL at all, and the surfactant may mainly act on lipoproteins other than sdLDL. Similarly, in the case of "surfactant that acts on (reacts with) LDLs other than sdLDL", the surfactant may mainly act on LDLs other than sdLDL. The term "elimination" means that a substance in a test sample is degraded so as to prevent the degraded product from being detected in the subsequent step. That is, the expression "eliminating cholesterol in lipoproteins other than sdLDL" means that lipoproteins other than sdLDL in the test sample are degraded so as to prevent the cholesterol in the lipoproteins, which is a degraded product, from being detected in the subsequent step. Similarly, the expression "eliminating cholesterol in LDLs other than sdLDL" means that cholesterol in LDLs other than sdLDL in the test sample is prevented from being detected in the subsequent step.

Specifically, the expression "led to the outside of the reaction system" means that cholesterol contained in HDL, VLDL, L LDL, and the like is eliminated or aggregated so as to prevent the cholesterol contained in HDL, VLDL, L LDL, and the like from affecting the quantification of sdLDL-C, or inhibited so as to avoid the reaction thereof in the subsequent step.

Hereinafter, the components contained in the first reagent composition will be described in more detail.

### (Polyoxyethylene Monostyrenated Phenyl Ether)

The first reagent composition contains polyoxyethylene monostyrenated phenyl ether (POE monostyrenated phenyl ether).

The POE monostyrenated phenyl ether may be composed of a plurality of compounds having different degrees of polymerization in POE moieties. At this time, the degree of polymerization n of polyoxyethylene in the POE monostyrenated phenyl ether is preferably 5 or more, and 80 or less from the viewpoint of improving accuracy in quantification of sdLDL-C. In addition, from the viewpoint of improving the accuracy in quantification of sdLDL-C, the average degree of polymerization of oxyethylene is, for example, more than 1, preferably 5 or more, and more preferably 10 or more, and for example, 100 or less, preferably 80 or less, more preferably 50 or less, and still more preferably 40 or less, and may be, for example, 30 or less.

Here, the average degree of polymerization is obtained from the intensity of the oxyethylene signal of the POE monostyrenated phenyl ether measured by NMR. For example, it can be calculated from a ratio of the signal intensity of the methylene protons (4 pieces) of the POE moiety of 3.5 ppm based on the intensity of the protons (9 pieces) of the TMS group of 0.08 ppm in the NMR spectrum of the TMS derivative of the POE monostyrenated phenyl ether.

In addition, from the viewpoint of more stably improving the accuracy in quantification of sdLDL-C, it is preferable that the first reagent composition contains one or more compounds selected from the group consisting of compounds in which the average degree of polymerization n of oxyethylene in the POE monostyrenated phenyl ether is n = 10 to 20.

Here, the degree of polymerization of oxyethylene of the component constituting the POE monostyrenated phenyl ether is obtained by structural analysis by liquid chromatography mass spectrometry. For example, when the POE monostyrenated phenyl ether in the first reagent composition are separated (from other materials) by a liquid chromatograph (LC) and then are introduced into a quadrupole time-of-flight mass spectrometer (QTOF mass spectrometer), a mass spectrum with peaks having an interval of 44 amu is obtained due to the difference in the degree of polymerization of oxyethylene of those components (with the same retention time). The degree of polymerization of oxyethylene of each component can be calculated by subtracting the mass number (MW 198) of monostyrenated phenol from the molecular weight estimated from the m/z of each peak and dividing the obtained value by 44. Further, the degree of polymerization of oxyethylene of each component can be analyzed by subtracting the chemical composition of monostyrenated phenol (C₁₄H₁₄O) from the compositional formula obtained from the accurate mass for each peak, or carrying out a detailed structural analysis by carrying out product ion scan.

From the viewpoint of improving the accuracy in quantification of sdLDL-C, the content of the POE monostyrenated phenyl ether in the first reagent composition is preferably 0.01% (w/v) or more, more preferably 0.03% (w/v) or more, still more preferably 0.05% (w/v) or more, and even still more preferably 0.1% (w/v) or more with respect to the entire first reagent composition.

In addition, from the same viewpoint, the content of the POE monostyrenated phenyl ether in the first reagent composition is preferably 0.6% (w/v) or less, more preferably 0.4% (w/v) or less, still more preferably 0.35% (w/v) or less, and even still more preferably 0.3% (w/v) or less with respect to the entire first reagent composition.

Here, the POE monostyrenated phenyl ether is available as an industrial raw material or a research reagent, and for example, a commercially available product can be used.

In addition, for example, POE monostyrenated phenyl ether may be produced and used. Specifically, the POE monostyrenated phenyl ether can be produced according to the following method and conditions.

That is, the POE monostyrenated phenyl ether is obtained by adding a predetermined amount of ethylene oxide to monostyrenated phenol in the presence of a basic catalyst. Examples of the basic catalyst include sodium hydroxide, potassium hydroxide, and alcoholate of an alkali metal. The addition reaction temperature is preferably 120°C to 200°C.

On the other hand, a styrenated phenol is obtained by carrying out an alkylation reaction of phenol and styrene at a temperature of 70°C to 200°C in the presence of an acid catalyst. As the acid catalyst, inorganic acids such as sulfuric acid and phosphoric acid, organic acids such as p-toluenesulfonic acid and methanesulfonic acid, Lewis acids such as a boron trifluoride diethyl ether complex, and the like can be used. In this reaction, by controlling conditions such as the equivalent ratio of phenol and styrene, the type of the acid catalyst, the reaction temperature, and the like, a monostyrenated phenol in which one styrene binds to the phenol and benzene ring can be obtained.

The monostyrenated phenol can be synthesized by, for example, adding styrene dropwise to phenol in the presence of a phosphoric acid catalyst to carry out an alkylation reaction, and then adding a sulfuric acid or magnesium sulfate catalyst in order to eliminate unreacted phenols and styrenes to complete the alkylation reaction. At this time, the amount of the phosphoric acid catalyst used is preferably 0.001 to 0.01 equivalents with respect to the phenol. The amount of the sulfuric acid or magnesium sulfate catalyst used is preferably 2% by mass to 10% by mass based on the mass of the phosphoric acid catalyst. In addition, the equivalent ratio of phenol and styrene is preferably 0.9 to 1.3 in terms of an equivalent ratio of the styrene with respect to the phenol.

Subsequently, after the alkylation reaction is completed, an aqueous solution such as sodium carbonate, potassium carbonate, sodium hydroxide, potassium hydroxide, or the like is added to neutralize the product of the alkylation reaction, and the produced neutralized salt is eliminated by filtration. Thus, the monostyrenated phenol can be recovered.

Then, in a pressure resistant container such as an autoclave, the POE monostyrenated phenyl ether can be obtained by carrying out an addition reaction of the obtained monostyrenated phenol and ethylene oxide under heating and pressure using potassium hydroxide as a catalyst. The heating and pressing conditions are, for example, a pressure of approximately 1.5 kg/cm³ and a temperature of approximately 130°C. In addition, by adjusting the molar ratio of ethylene oxide with respect to 1 mol of monostyrenated phenol, the number of repeating units of the oxyethylene unit, that is, the degree of polymerization of oxyethylene can be adjusted.

In addition, in the above method, a mixture of a monostyrenated phenol, a distyrenated phenol to which two styrenes are bound, and a tristyrenated phenol to which three styrenes are bound is obtained as the styrenated phenol, and the monostyrenated phenol can be separated and purified from the mixture and used.

The POE monostyrenated phenyl ether in the first reagent composition can be confirmed by, for example, a method of carrying out analysis by combining IR, NMR, LC-MS and the like. Examples of a method of determining the structure of the POE monostyrenated phenyl ether in the first reagent composition include method of carrying out analysis using LC/MS/MS and NMR.

In addition, the concentration of the POE monostyrenated phenyl ether in the first reagent composition can be calculated, for example, by HPLC or LC/MS measurement. In addition, the concentration of the POE monostyrenated phenyl ether in the first reagent composition can be obtained by NMR measurement.

### (Enzyme)

The first reagent composition has one or two or more activities selected from the group consisting of cholesterol esterase (CHE) activity, cholesterol oxidase (COO) activity, and sphingomyelinase activity.

From the viewpoint of causing the first reagent composition to stably act on lipoproteins other than sdLDL and leading the cholesterol to the outside of the reaction form, preferably, the first reagent composition further has at least one activity selected from the group consisting of peroxidase activity (POD) and catalase activity.

Here, the expression "having cholesterol esterase activity" specifically refers to that an enzyme (for example, cholesterol esterase) having the ability to hydrolyze cholesterol ester, which is a substrate, is present, and a reaction catalyzed by cholesterol esterase may occur. The same applies to other enzyme activities such as cholesterol oxidase activity.

The expression "having sphingomyelinase activity" refers to that an enzyme having the ability to degrade sphingomyelin, which is a substrate, is present, and a reaction catalyzed by the enzyme may occur. The enzymes having the sphingomyelinase activity include sphingomyelinase C and sphingomyelinase D, as well as phospholipase C and phospholipase D having the ability to degrade sphingomyelin.

In addition, the first reagent composition specifically contains at least one enzyme selected from the group consisting of an enzyme having cholesterol esterase activity, an enzyme having cholesterol oxidase activity, and an enzyme having sphingomyelinase activity, and more specifically contains at least one selected from the group consisting of cholesterol esterase, cholesterol oxidase, and sphingomyelinase.

From the viewpoint of causing the first reagent composition to stably act on lipoproteins other than sdLDL and leading the cholesterol to the outside of the reaction form, the first reagent composition preferably further contains at least one enzyme selected from the group consisting of an enzyme having peroxidase activity and an enzyme having catalase activity, and more preferably specifically contains at least one of peroxidase and catalase.

As each of the cholesterol esterase, the cholesterol oxidase, the sphingomyelinase, the peroxidase, and the catalase, for example, those derived from bacteria or fungi or those derived from plants can be used.

From the viewpoint of more stably leading cholesterol in lipoproteins other sdLDL to the outside of the reaction system, the cholesterol esterase activity of the first reagent composition is preferably 50 U/L or more and more preferably 100 U/L or more, and is preferably 3000 U/L or less, more preferably 2500 U/L or less, still more preferably 2000 U/L or less, even still more preferably 1000 U/L or less. The cholesterol esterase activity may be, for example, 1800 U/L or less, or, for example, 1500 U/L or less.

From the viewpoint of more stably leading cholesterol in lipoproteins other sdLDL to the outside of the reaction system, the cholesterol oxidase activity of the first reagent composition is preferably 100 U/L or more and more preferably 150 U/L or more, and is preferably 800 U/L or less, more preferably 750 U/L or less, still more preferably 700 U/L or less, even still more preferably 650 U/L or less, and even still more preferably 600 U/L or less.

From the viewpoint of more stably leading cholesterol in lipoproteins other sdLDL to the outside of the reaction system, the sphingomyelinase activity of the first reagent composition is preferably 100 U/L or more and more preferably 200 U/L or more, and is preferably 3000 U/L or less and more preferably 2800 U/L or less.

From the viewpoint of more stably leading cholesterol in lipoproteins other sdLDL to the outside of the reaction system, the peroxidase activity of the first reagent composition is preferably 200 U/L or more and more preferably 300 U/L or more, is preferably 3000 U/L or less, more preferably 2500 U/L or less, and is also preferably, for example, 2000 U/L or less. In addition, the peroxidase activity of the first reagent composition may be, for example, 5000 U/L or less or, for example, 4000 U/L or less.

From the viewpoint of stably eliminating hydrogen peroxide generated when the first reagent composition is applied to the sample, and the viewpoint of more stably leading cholesterol in lipoproteins other sdLDL in the sample to the outside of the reaction system, the catalase activity of the first reagent composition is preferably 100 KU/L or more and more preferably 200 KU/L or more, and is preferably 2000 KU/L or less and more preferably 1500 KU/L or less.

In the first reagent composition, each of the cholesterol oxidase activity, cholesterol esterase activity, sphingomyelinase activity, peroxidase activity, and catalase activity can be measured by, for example, the following method.

In the measurement of the cholesterol oxidase activity, a 6 mM cholesterol solution (the cholesterol is dissolved in isopropanol) is used as a substrate solution. A dilution solution (0.1 M phosphate buffer solution, Triton X100, pH 7.0) is added so that the concentration of the measurement target is 2 to 4 U/mL, 3 mL of the dilution solution is heated at 37°C for 5 minutes, and 0.05 mL of a substrate solution is then added thereto. Then, the mixed solution is allowed to react at 37°C and the amount of change in absorbance at a wavelength of 240 nm is measured. After the reaction at 37°C, the amount of change in absorbance is measured from 2 minutes to 7 minutes, and the cholesterol oxidase activity is calculated. For example, in a case where the amount of change in absorbance is 3 U/L or more, it can be said that the measurement target has cholesterol oxidase activity, and more specifically, it can be said that cholesterol oxidase is contained.

In the measurement of the cholesterol esterase activity, a substrate (a solution of 0.04% cholesterol linolenic acid, 1% Triton X100, and 0.6% sodium cholate), a solution of 300 U/mL cholesterol oxidase, an enzyme dilution solution (a 20 mM phosphate buffer solution containing 0.5 mM EDTA·2Na, 2 mM MgCl₂, and 0.2% bovine serum albumin (BSA), pH 7.5), and a reaction solution (0.06% 4-aminoantipyrine, 0.4% phenol, 7.5 KU/L peroxidase (POD)) are used. After mixing 1.75 mL of the reaction solution and 1.0 mL of the substrate solution, the mixed solution is heated at 37°C for 5 minutes, and a 0.1 mL cholesterol oxidase solution is added thereto. After heating the mixed solution at 37°C for 2 minutes, 0.1 mL of the measurement target diluted with the dilution solution is added, the mixed solution is allowed to react at 37°C, and the amount of change in absorbance at a wavelength of 500 nm is measured. After the reaction at 37°C, the amount of change in the absorbance from 0 minutes to 3.5 minutes is measured, and the cholesterol esterase activity is calculated. For example, when the amount of change in the absorbance is 8 U/L or more, it can be said that the measurement target has cholesterol esterase activity, and more specifically, it can be said that cholesterol esterase is contained.

The sphingomyelinase activity of the first reagent composition is measured, for example, by the following method. That is, a reaction solution (a mixed solution of 0.008% sphingomyelin, a 0.05% Triton X100 solution, 10 U/mL alkaline phosphatase, 10 U/mL cholesterol oxidase, 2 U/mL peroxidase, 0.02% 4-aminoantipyrine, and 0.02% TODB), a reaction stopping solution (a 1% sodium dodecyl sulfate solution), and a dilution solution (a 10 mM Tris buffer solution containing 0.1% Triton X100, pH 8.0) are used. 0.08 mL of the reaction solution and 0.003 mL of the measurement target diluted with the dilution solution are mixed, the mixed solution is heated at 37°C for 5 minutes, and then 0.16 mL of the reaction stopping solution is added thereto. After stopping the reaction, the amount of change in the absorbance at a main wavelength of 546 nm and a minor wavelength of 700 nm is measured to calculate the sphingomyelinase activity. For example, in a case where the amount of change in absorbance is 2 U/L or more, it can be said that the measurement target has sphingomyelinase activity, and more specifically, it can be said that the measurement target contains sphingomyelinase.

The peroxidase activity of the first reagent composition is measured, for example, by the following method. That is, a reaction solution 1 (1.5 mM HDAOS, 0.05% Triton X100, and 50 mM phosphate buffer solution, pH 7.0) and reaction solution 2 (5 mM 4-aminoantipyrine, 0.05% Triton X100, 1% hydrogen peroxide, and 50 mM phosphate buffer solution, pH 7.0), and a dilution solution (50 mM phosphate buffer solution, pH 7.0) are used. 0.3 mL of the reaction solution 1 and 0.08 mL of the measurement target diluted with the dilution solution are mixed and the mixed solution is heated at 37°C for 5 minutes. Then, 0.1 mL of the reaction solution 2 is added thereto, the resultant mixture is allowed to react at 37°C, and the amounts of change in the absorbance at a main wavelength of 600 nm and a minor wavelength of 700 nm are measured. After the reaction at 37°C, the amount of change in the absorbance from 2 minutes to 5 minutes is measured to calculate the peroxidase activity. For example, in a case where the amount of change in the absorbance is 10 U/L or more, it can be said that the measurement target has peroxidase activity, and more specifically, it can be said that the measurement target contains peroxidase.

The catalase activity of the first reagent composition is measured, for example, by the following method. That is, in the measurement of the catalase activity, a substrate (0.06% hydrogen peroxide, and 50 mM phosphate buffer solution, pH 7.0) is used. After preheating 2.0 mL of the substrate solution at 25°C, the substrate solution is mixed with 0.1 mL of a measurement target, and the amount of change in the absorbance at 240 nm is measured. For example, after the reaction at 25°C, the amount of change in the absorbance from 0 minutes to 3 minutes is measured to calculate the catalase activity. For example, in a case where the amount of change in the absorbance is 50 U/L or more, it can be said that the measurement target has a catalase activity, and more specifically, it can be said that the measurement target contains catalase.

The first reagent composition may further have other enzyme activities, and specifically, one or two or more enzyme activities selected from the group consisting of ascorbate oxidase activity and lipoprotein lipase (LPL) activity.

In addition, the first reagent composition may contain, for example, an enzyme having one or two or more enzyme activities selected from the group consisting of an enzyme having ascorbate oxidase activity and an enzyme having lipoprotein lipase activity, and specifically contains one or two or more enzymes selected from the group consisting of ascorbate oxidase and lipoprotein lipase.

From the viewpoint of avoiding the influence of ascorbic acid coexisting in the sample on the measurement accuracy, the first reagent composition preferably further has ascorbate oxidase activity and is preferably contains an enzyme having ascorbate oxidase activity and more preferably contains ascorbate oxidase.

From the viewpoint of avoiding the influence of ascorbic acid coexisting in the sample on the measurement accuracy, the ascorbate oxidase activity of the first reagent composition is preferably 0.1 U/mL or more and more preferably 0.2 U/mL or more, and is preferably 15 U/mL or less and more preferably 10 U/mL or less.

The ascorbate oxidase activity of the first reagent composition is measured, for example, by the following method. That is, in the measurement of the ascorbate oxidase activity, a substrate (10 mM ascorbic acid solution -0.13 mM EDTA is diluted 20 times with 90 mM KH₂PO₄ - 5 mM NaHPO₄) is used. 1 mL of the substrate is preheated at 30°C, and after 5 minutes, 0.1 mL of the measurement target diluted with a 90 mM NaHPO₄ solution containing 0.05% BSA is added and mixed to initiate the reaction. After 5 minutes, 3.0 mL of a reaction stopping solution (0.2N HCl) is added to stop the reaction, and then the absorbance at 245 nm is measured to calculate the ascorbate oxidase activity. For example, in a case where the activity amount is 10 U/L or more, it can be said that the measurement target has ascorbate oxidase activity, and more specifically, it can be said that the measurement target contains "ascorbate oxidase".

In addition, from the viewpoint of preferably adjusting the action on various lipoproteins, the first reagent composition further has lipoprotein lipase activity, preferably contains an enzyme having lipoprotein lipase activity, and more preferably contains lipoprotein lipase.

In addition, in the present embodiment, the enzymes in the first reagent composition and the second reagent composition described later can also be identified by the following method. That is, first, a fragment peptide obtained by degrading a sample containing a target enzyme with trypsin is detected by a hybrid mass spectrometer. Proteins can be identified by database search (for example, Mascot search) of the mass of peptides obtained using a mass spectrometer and the spectrum (MS/MS data) of fragment ions obtained by colliding with argon gas in the mass spectrometer. When the sequence of the fragment peptide derived from the amino acid sequence in the reagent composition matches with the amino acid sequence registered in the database as a unique sequence, it can be considered that the target enzyme is contained.

The enzymes in the first reagent composition and a second reagent composition described later can be identified, for example, by the following quantification. That is, among the fragment peptides obtained by degrading the target enzyme with trypsin, a peptide that is specific to the target enzyme and gives a strong signal in mass spectrometry is selected as a peptide to be quantified. For the peptide to be quantified, an unlabeled peptide and a peptide labeled with a stable isotope as an internal standard are prepared by chemical synthesis. A sample containing the target enzyme is completely digested with trypsin, a known amount of stable isotope-labeled peptide is added, and the measurement is carried out with a triple quadrupole mass spectrometer (LC-MS/MS) connected to HPLC, in a multiple reaction monitoring mode (MRM mode). A mixed solution of the unlabeled peptide of the peptide to be quantified and the known amount of stable isotope-labeled peptide is measured in the same manner to create a calibration curve of the concentration ratio and the peak area ratio of the internal standard, and the absolute amount of the peptide to be quantified in the sample is calculated. Thus, the target enzyme can be quantified.

### (Other Components)

The first reagent composition may contain components other than those described above. Examples of the other components include a buffer solution, a salt, a protein having no enzymatic action, a preservative, a hydrogen donor, a coupler, and a surfactant other than the polyoxyethylene monostyrenated phenyl ether.

The type of buffer solution can be appropriately selected, for example. Specific examples of the buffer solution include a MOPS (3-morpholinopropane sulfonic acid) buffer solution, a phosphate buffer solution, a Tris buffer solution, a PIPES (piperazine-1,4-bis(2-ethanesulfonic acid)) buffer solution, and a HEPES (4-(2-hydroxyethyl)-1-piperazineethanesulfonic acid) buffer solution.

From the viewpoint of maintaining the enzyme activity in the composition and the viewpoint of improving the storage stability of the reagent, the concentration of the buffer solution is preferably 1 mM or more, more preferably 5 mM or more, and still more preferably 10 mM or more, and is preferably 300 mM or less, more preferably 200 mM or less, still more preferably 150 mM or less, and even still more preferably 100 mM or less.

Specifically, a salt is formulated as a pH adjuster or an ionic strength adjuster. Specific examples of the salt include basic substances such as sodium salts such as sodium hydroxide and sodium sulfate; potassium salts such as potassium hydroxide; magnesium salts such as magnesium chloride and magnesium sulfate; and ammonium salts such as ammonium sulfate and ammonium chloride. In addition, when the first reagent composition contains at least one of a monovalent cation and a divalent cation, or a salt thereof, the fractionation of sdLDL and L LDL becomes easier.

From the viewpoint of causing the salt to act more stably as a pH adjuster or an ionic strength adjuster, the concentration of the salt in the first reagent composition is preferably 2 mmol/L or more and more preferably 5 mmol/L or more, and is preferably 50 mmol/L or less and more preferably 30 mmol/L or less with respect to the total composition of the first reagent composition.

From the viewpoint of maintaining the enzyme activity in the composition and the viewpoint of improving the storage stability of the reagent, the pH of the first reagent composition is preferably 6.0 or more and more preferably 6.5 or more, and is preferably 8.0 or less and more preferably 7.5 or less.

Specific examples of the protein having no enzymatic action include albumin such as bovine serum albumin (BSA).

From the viewpoint of stabilizing the enzyme in the first reagent composition and the viewpoint of stabilizing the first step of leading the cholesterol from lipoproteins other than sdLDL to the outside of the reaction system, the concentration of albumin such as BSA in the first reagent composition is preferably 1 g/L or more and more preferably 2 g/L or more, and is preferably 20 g/L or less and more preferably 10 g/L or less with respect to the total composition of the first reagent composition.

The first reagent composition preferably contains at least one of a hydrogen donor and a coupler, and more preferably contains any one of a hydrogen donor and a coupler. At this time, any one of a hydrogen donor and a coupler is used in the first step to lead cholesterol in lipoproteins other than sdLDL to the outside of the reaction system. More specifically, any one of the hydrogen donor and the coupler is used in order to cause the cholesterol esterase or the cholesterol oxidase to act on the cholesterol in the lipoproteins other than sdLDL and convert the generated hydrogen peroxide to colorless quinone in the presence of the peroxidase.

In addition, from the viewpoint of improving the preserving property of the first reagent composition, the first reagent composition preferably contains any one of a hydrogen donor and a coupler, and more preferably contains a hydrogen donor and does not contain a coupler, and still more preferably contains a hydrogen donor and a peroxidase and does not contain a coupler.

On the other hand, from the viewpoint of easily confirming the reactivity of the first reagent composition in the first step described later, the first reagent composition preferably contains a hydrogen donor and a coupler, and more preferably contains a hydrogen donor, a coupler, and peroxidase. From the same viewpoint, it is also preferable that the first reagent composition contains a coupler and peroxidase.

Specific examples of the hydrogen donor include aniline derivatives such as N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), N-(3-sulfopropyl)-3-methoxy-5-aniline (HMMPS), N-ethyl-N-(2-hydroxy-3-sulfopropyl)-4-fluoro-3,5-dimethoxyaniline (FDAOS), and N-ethyl-N-(3-methylphenyl)-N'-succinylethylenediamine (EMSE) .

From the viewpoint of leading cholesterol in lipoproteins other than sdLDL to the outside of the reaction system, the concentration of the hydrogen donor in the first reagent composition is preferably 1 mM or more and more preferably 1.5 mM or more, and is preferably 5 mM or less and more preferably 3 mM or less.

In addition, in a case where the hydrogen donor is contained in the second reagent composition described later, the first reagent composition preferably contains a coupler that is used for the coupling reaction. As the coupler, for example, 4-aminoantipyrine (4AA), an aminoantipyrine derivative, vanillindiamine sulfonic acid, methylbenzthiazolinone hydrazone, sulfonated methylbenzthiazolinone hydrazone, and the like can be used, and the coupler is not limited thereto.

The concentration of the coupler in the first reagent composition is preferably 0.2 mM or more, and more preferably 0.3 mM or more, and is preferably 5.0 mM or less and more preferably 3.3 mM or less with respect to the total composition of the reaction solution after the addition of the first reagent composition from the viewpoint of causing the coupler to stably act on sdLDL.

In the present embodiment, since the first reagent composition has one or two or more activities selected from the group consisting of cholesterol esterase activity, cholesterol oxidase activity, and sphingomyelinase activity, and contains polyoxyethylene monostyrenated phenyl ether, the first reagent composition effectively acts on LDLs other than sdLDL and decreases the action on sdLDL, has excellent selectivity for LDL other than sdLDL, and can be suitably used for quantification of sdLDL-C. Therefore, by using the first reagent composition in the present embodiment, excellent accuracy can be obtained in the measurement of sdLDL-C.

In the present embodiment, the first reagent composition is used for quantifying sdLDL-C, for example, in combination with a second reagent composition described later.

### (Kit)

In the present embodiment, the kit is used for quantifying sdLDL-C in a sample, and contains the above-mentioned first reagent composition and a second reagent composition for quantifying sdLDL-C.

In addition, the kit is specifically used for a method of quantifying sdLDL-C including two or more steps. At this time, the first and second reagent compositions are used in different steps, and the first and second reagent compositions are preferably used in this order.

Hereinafter, the configuration of the second reagent composition will be described in more detail.

### (Second Reagent Composition)

The second reagent composition is specifically a reagent composition for quantifying sdLDL-C. Although the components of the second reagent composition differ depending on the configuration of the first reagent composition, any formulation composition capable of quantifying sdLDL-C may be used, and known substances can be used.

Specific examples of the component contained in the second reagent composition include an enzyme, a buffer solution, a salt, a surfactant, a protein having no enzymatic action, a preservative, and any one of a hydrogen donor and a coupler.

In addition, in a preferable configuration of the kit, the first reagent composition contains any one of a hydrogen donor and a coupler and does not contain the other, and the second reagent composition does not contain any one of the hydrogen donor and the coupler and contains the other.

Examples of the enzyme include peroxidase. In addition, for example, the second reagent composition has peroxidase activity. From the viewpoint of accurately measuring sdLDL-C in the second step, the peroxidase activity of the second reagent composition is preferably 500 U/L or more and more preferably 1000 U/L or more, and is preferably 10000 U/L or less. However, since the peroxidase activity is carried over to the second step in a case where the peroxidase activity is contained in the first reagent composition, it is also possible to reduce the concentration or make the peroxidase not contained in the second reagent.

The types of buffer solution and salt can be appropriately selected depending on, for example, the type of enzyme contained in the second reagent composition. Specific examples of the buffer solution include those described above for the first reagent composition.

From the viewpoint of maintaining the enzyme activity in the composition and the viewpoint of improving the storage stability of the reagent, the pH of the second reagent composition is preferably 6.0 or more and more preferably 6.5 or more, and is preferably 8.0 or less and more preferably 7.5 or less.

Examples of the surfactant include surfactants that act on sdLDL. In addition, the second reagent composition preferably contains a surfactant that acts on sdLDL from the viewpoint of stably quantifying sdLDL-C.

The surfactant that acts on sdLDL may be a surfactant that selectively acts on sdLDL, such as a surfactant that acts only on sdLDL, or a surfactant that also acts on lipoproteins other than sdLDL, or a surfactant that acts on all lipoproteins.

As the surfactant in the second reagent composition, for example, a polyoxyethylene derivative may be mentioned, and any commercially available surfactant can be used as long as the surfactant is used in reagents or the like for total cholesterol measurement. Examples of such a surfactant include polyoxyethylene alkyl phenyl ether such as polyoxyethylene octylphenyl ether (for example, Emulgen 909 (manufactured by Kao Corporation), and Triton X-100) and polyoxyethylene alkyl ether (for example, Emulgen 707 and Emulgen 709 (manufactured by Kao Corporation)).

The concentration of the surfactant in the second reagent composition is preferably 0.05% (w/v) or more, more preferably 0.1% (w/v) or more, and still more preferably 0.5% (w/v) or more with respect to the mixed solution after the addition of the second reagent composition from the viewpoint of causing the surfactant to stably act on sdLDL.

In addition, from the same viewpoint, the concentration of the surfactant in the second reagent composition is preferably 8.0% (w/v) or less, and more preferably 5.0% (w/v) or less.

When the second reagent composition contains a coupler, the coupler is preferably one or two or more compounds selected from the group consisting of 4-aminoantipyrine (4AA), an aminoantipyrine derivative, vanillindiamine sulfonic acid, methylbenzthiazolinone hydrazone, and sulfonated methylbenzthiazolinone hydrazone.

The concentration of the coupler in the second reagent composition is preferably 0.5 mM or more, and more preferably 1.0 mM or more, and is preferably 15 mM or less and more preferably 10 mM or less with respect to the total composition of the reaction solution after the addition of the second reagent composition from the viewpoint of causing the coupler to stably act on sdLDL.

On the other hand, in a case where the coupler is contained in the first reagent composition, the hydrogen donor is preferably contained in the second reagent composition. In this case, the concentration of the hydrogen donor in the reagent is preferably 3 mM or more, and more preferably 4.5 mM or more, and is preferably 15 mM or less and more preferably 12 mM or less with respect to the total composition of the reaction solution after the addition of the second reagent composition from the viewpoint of causing the hydrogen donor to stably act on sdLDL.

Specific examples of the protein having no enzymatic action include those described above for the first reagent composition.

### (Method)

A method in the present embodiment is a method of quantifying sdLDL-C in a sample using the above-mentioned first and second reagent compositions. The quantification method in the present embodiment includes the following first step and second step.

(First Step) Causing the above-mentioned first reagent composition to act on a sample.

(Second Step) After the first step, applying the above-mentioned second reagent composition to quantify cholesterol in the remaining lipoprotein.

The configurations of the first and second reagent compositions are as described above. In such a method, by using the first reagent composition according to the present embodiment, sdLDL-C can remain in the reaction solution in the first step with high selectivity, and thus sdLDL-C can be measured with excellent accuracy. Therefore, for example, sdLDL-C can be stably quantified with high accuracy.

In addition, the method of quantifying sdLDL-C may be carried out by, for example, adding the first reagent composition to a sample (test sample) to allow a reaction to proceed, then adding the second reagent composition to the sample to allow a reaction to proceed, and measuring the absorbance.

The test sample is, for example, a sample derived from blood such as serum or plasma, and preferably serum.

The first and second steps are usually carried out in an automated analysis device.

The amount of the sample and the amount of each reagent composition can be appropriately determined in consideration of, for example, the concentration of the reagent in each reagent composition or the like, and the steps are carried out within the range applicable to the automated analysis device. For example, 1 to 10 µL of the test sample, 50 to 300 µL of the first reagent, and 25 to 200 µL of the second reagent may be used.

Each step will be described in more detail below.

### (First Step)

In the first step, the first reagent composition is allowed to act on a sample. By doing this, lipoproteins other than sdLDL are eliminated, and cholesterol in the lipoproteins other than sdLDL is liberated and led to the outside of the reaction system.

More specifically, in the first step, preferably, a surfactant that acts on lipoproteins other than the sdLDL is allowed to act on the sample in the presence of cholesterol esterase. Then, cholesterol generated by liberation from lipoproteins is allowed to react with an enzyme that reacts with cholesterol, such as cholesterol oxidase, and is led to the outside of the reaction system. In the first step, for example, known techniques for eliminating cholesterol in lipoproteins other than sdLDL and leading the cholesterol to the outside of the reaction system, aggregating cholesterol in lipoproteins other than sdLDL, inhibiting cholesterol in lipoproteins other than sdLDL to avoid the reaction in the subsequent step, and the like can be used.

When the first reagent composition has an electron donor, eliminating cholesterol generated from lipoproteins other than sdLDL and leading the cholesterol to the outside of the reaction system in the first step may include, for example, forming hydrogen peroxide generated by cholesterol esterase activity and cholesterol oxidase activity in the first reagent composition, and a colorless quinone in the presence of an electron donor.

In addition, in the first step, at least one of a monovalent cation and a divalent cation or a salt thereof can be used as an ionic strength adjuster by further adding such ionic strength adjuster to the reaction solution. Addition of such ionic strength adjuster facilitates differentiation of sdLDL and L LDL.

### (Second Step)

In the second step, the sdLDL-C remaining after the first step is quantified. In the second step, an LDL quantification method used in the related art can be used. Examples thereof include a method of quantifying the content of an LDL specific aggregate formed by adding an LDL aggregating agent according to turbidimetry, a method of using an antigen-antibody reaction with an LDL specific antibody, a method of quantifying a degraded product using an enzyme. The quantification method is selected, for example, according to components contained in or a formulation of the second reagent composition.

When the second reagent composition contains an enzyme, a method of quantifying a degraded product using an enzyme can be adopted. Specifically, the second reagent composition containing, for example, one or two or more enzymes for measuring cholesterol, selected from the group consisting of cholesterol esterase, cholesterol oxidase, cholesterol dehydrogenase, and peroxidase is added to the reaction solution after the first step, to liberate and degrade sdLDL-C and quantifying the reaction product thereof.

At this time, the second reagent composition preferably contains the above-described surfactant.

In the present embodiment, the reaction temperature in each step is preferably 2°C to 45°C, and more preferably 25°C to 40°C.

Regarding the reaction time, each step is preferably carried out for 1 to 10 minutes and more preferably carried out for 3 to 7 minutes.

Examples of the automated analysis device to be used for quantifying sdLDL-C include TBA-120FR and TBA-200FR (all manufactured by TOSHIBA Corporation), JCA-BM 1250, JCA-BM 1650, and JCA-BM 2250 (all manufactured by JEOL Ltd.), HITACHI 7180, and HITACHI 7170 (all manufactured by Hitachi, Ltd.), AU2700, AU5800, and AU680 (all manufactured by Olympus Corporation), and cobas c501 and cobas c701 (all manufactured by Roche).

The sdLDL-C is quantified by measuring the absorbance in a wavelength range of 580 to 720 nm, preferably 600 to 700 nm, for example.

Although the embodiments of the present invention have been described above, these are examples of the present invention, and various configurations other than the above can be adopted.

### Examples

In the following, "%" of the POE monostyrenated phenyl ether concentration is specifically "% (w/v)".

### (Experimental Example 1)

### (Examples 1 to 3 and Comparative Example 1)

In order to evaluate the reactivity of the surfactant of each example in the sdLDL-C reagent, the following reagent was prepared and measurement was carried out.

### (Preparation of Reagent Composition)

Each component shown below was formulated at the following concentration, and the type of surfactant was changed to prepare the first reagent composition of each example. The formulation composition of the first reagent composition is shown below.

### (First Reagent Composition)

| | |
|---|---|
| PIPES buffer solution, pH 7.0 | 50 mM |
| Cholesterol esterase | 900 U/L |
| Cholesterol oxidase | 450 U/L |
| Sphingomyelinase | 525 U/L |
| Peroxidase | 1250 U/L |
| Bovine serum albumin | 0.75% (w/v) |
| TOOS | 1.5 mM |
| 4AA | 1 mM |
| Surfactant *1 | |

| | |
|---|---|
| [0083] *1 Surfactant | |

Example 1: POE monostyrenated phenyl ether, a degree of polymerization of POE of 11 to 33
Example 2: POE monostyrenated phenyl ether, a degree of polymerization of POE of 13 to 37
Example 3: POE monostyrenated phenyl ether, a degree of polymerization of POE of 50 to 80
Comparative Example 1: Mixture of POE distyrenated phenyl ether, a degree of polymerization of POE of 2 to 32, and POE tristyrenated phenyl ether, a degree of polymerization of POE of 5 to 30

### (Evaluation of Lipoprotein Selectivity)

In order to evaluate the lipoprotein selectivity in the reaction with the first reagent composition, the reactivity of sdLDL and lbLDL with the above-mentioned reagent composition was measured, respectively.

Specifically, sdLDL and lbLDL separated from human serum by ultracentrifugation were used as samples. In order to confirm the reactivity of the surfactant with respect to the sample, the first reagent composition containing cholesterol esterase, cholesterol oxidase, which are components involved in the color reaction, as well as peroxidase, a hydrogen donor, and a coupler, was prepared. 3 µL of the sample and 150 µL of the first reagent composition were mixed, and a difference in absorbance at 600 nm (main wavelength) and 700 nm (minor wavelength) after 5 minutes at 37°C (in Table 1 and Table 2 to be described later, "absorbance at wavelength 600 nm/700 nm") [mAbs] was measured to obtain the ratio of the absorbance of lbLDL with respect to the absorbance of sdLDL. The measurement results are shown in Table 1.

**[Table 1]**

| | Example 1 | Example 2 | Example 3 | Comparative Example 1 |
|---|---|---|---|---|
| POE monostyrenated phenyl ether (%) | 0.17 | 0.17 | 0.34 | 0 |
| lbLDL absorbance (at wavelength 600/700 nm) [mAbs] | 256.3 | 235.2 | 177.7 | 157.2 |
| sdLDL absorbance (at wavelength 600/700 nm) [mAbs] | 176.1 | 168.3 | 99.1 | 149.8 |
| lb/sd absorbance ratio | 1.46 | 1.4 | 1.79 | 1.05 |

As seen from Table 1, in each of Examples containing the POE monostyrenated phenyl ether, compared with Comparative Examples containing POE distyrenated phenyl ether and POE tristyrenated phenyl ether and not containing POE monostyrenated phenyl ether, the absorbance ratio of lbLDL/sdLDL is large, and the selectivity of the first reagent composition to lbLDL is excellent.

Therefore, by using the first reagent composition of each of Examples containing POE monostyrenated phenyl ether as the surfactant, sdLDL in the sample can be quantified with higher accuracy.

### (Experimental Example 2)

The absorbance ratio of lbLDL/sdLDL was obtained in the same manner as in Experimental Example 1 except that the POE monostyrenated phenyl ether concentration in Example 1 was changed. The concentration at which the lbLDL/sdLDL ratio was 1.2 or more was defined as the acceptable concentration.

The measurement results are shown in Table 2.

**[Table 2]**

| | | | | | | |
|---|---|---|---|---|---|---|
| POE monostyrenated phenyl ether (%) | 0.04 | 0.08 | 0.17 | 0.34 | 0.5 | 0.63 |
| lbLDL absorbance (at wavelength 600/700 nm) [mAbs] | 347.9 | 318.3 | 256.3 | 188 | 135.2 | 122.3 |
| sdLDL absorbance (at wavelength 600/700 nm) [mAbs] | 278.5 | 235.2 | 176.1 | 125.5 | 93 | 83 |
| lb/sd absorbance ratio | 1.25 | 1.35 | 1.46 | 1.5 | 1.45 | 1.47 |

As seen from Table 2, the selectivity of the first reagent composition to lbLDL is excellent at each concentration.

### (Experimental Example 3)

A first reagent composition was prepared by using POE monostyrenated phenyl ether used in Example 1 as a surfactant and changing the concentration thereof. The accuracy at the measurement of sdLDL-C of a sample having a known concentration with a second reagent composition was evaluated by a comparative method. Specifically, the following first reagent composition having different concentrations of the surfactant used in Example 1 and the following second reagent composition were prepared. 75 µL of the first reagent composition was added to 2 µL of the serum sample and allowed to react at 37°C for 5 minutes, then, 75 µL of the second reagent composition is added thereto and allowed to react for 5 minutes to measure the absorbance at a main wavelength of 600 nm and a minor wavelength of 700 nm. Then, the concentration of sdLDL-C was calculated from a calibration curve created separately. The formulation compositions of the first reagent composition and the second reagent composition are shown below.

### (First Reagent Composition)

| | |
|---|---|
| PIPES buffer solution, pH 7.0 | 50 mM |
| Cholesterol esterase | 300 U/L |
| Cholesterol oxidase | 600 U/L |
| Sphingomyelinase | 2700 U/L |
| Catalase | 1200 KU/L |
| Ascorbate oxidase | 3000 U/L |
| Bovine serum albumin | 10 g/L |
| TOOS | 2.0 mM |
| Surfactant *1 | |
| 0.05%, 0.07%, 0.09%, 0.11%, 0.13%, 0.16%, 0.18%, 0.20%, or 0.30% | |

| (Second Reagent Composition) | |
|---|---|
| PIPES buffer solution, pH 7.0 | 50 mM |
| 4-aminoantipyrine | 4.0 mM |
| Peroxidase | 5000 U/L |
| Sodium azide | 0.05% (w/v) |
| Polyoxyethylene alkyl ether | 1% (w/v) |

By using the sdLDL-C value obtained under the following conditions by an ultracentrifugation method, specifically, a method of fractionating sdLDL by ultracentrifugation and measuring cholesterol, as the comparative method, the correlation coefficient with the concentration of sdLDL-C calculated from the above-mentioned calibration curve was calculated.

In the ultracentrifugation method, a fraction having a density of 1.044 to 1.063 g/cm³ corresponding to sdLDL was separated by the method described in clinical chemistry, 57, p. 57 to 65, 2011, and the concentration of sdLDL-C was measured.

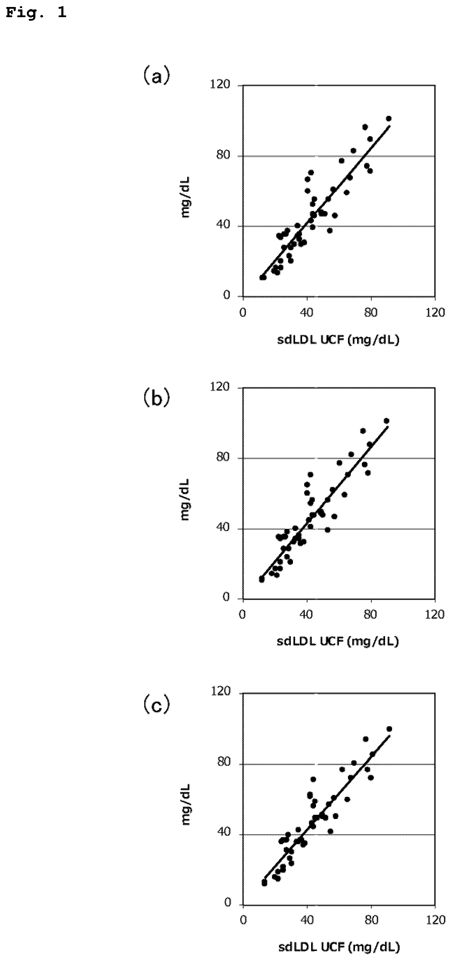

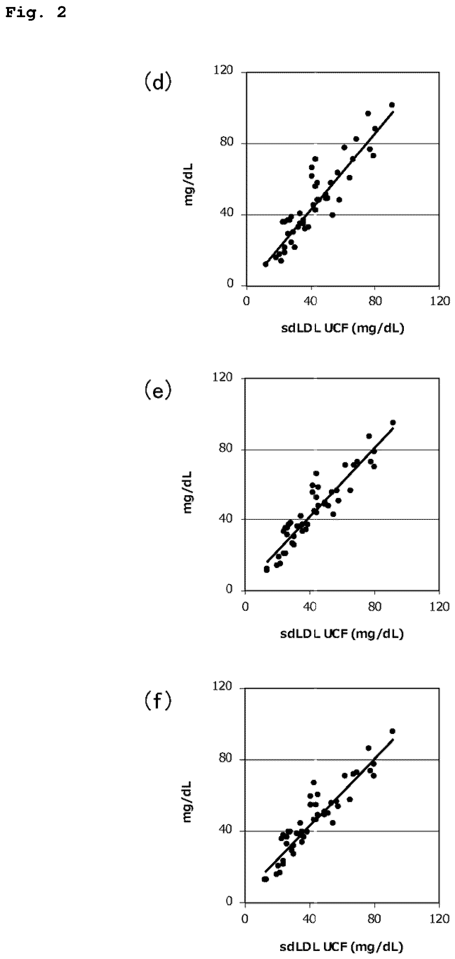

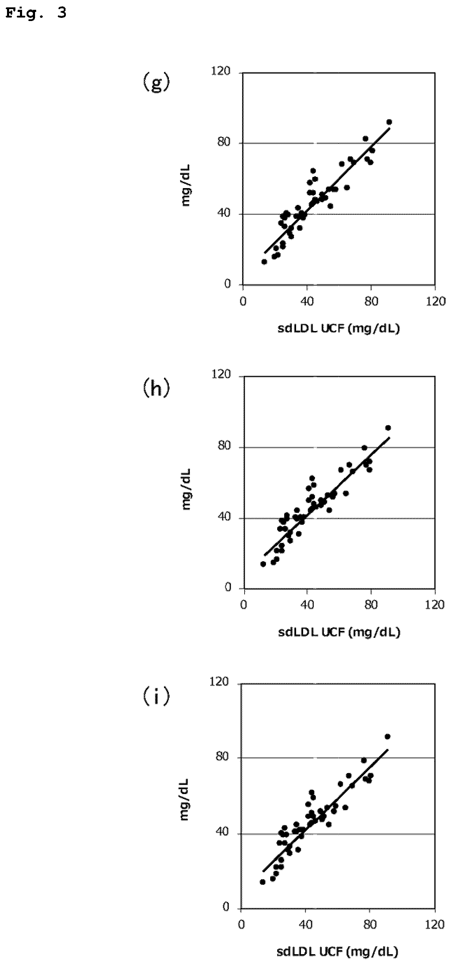

The evaluation results are shown in Figs. 1(a) to 1(c), 2(d) to 2(f), and 3(g) to 3(i). These are diagrams showing the evaluation results of the accuracy of the measured value of the concentration of sdLDL-C, the horizontal axis represents the measured value obtained by the ultracentrifugation (UCF) method, and the vertical axis represents the measured value calculated from the above-mentioned calibration curve.

In addition, the concentrations of POE monostyrenated phenyl ether and the correlation coefficients in Figs. 1(a) to 1(c),2(d) to 2(f), and 3(g) to 3(i) are shown below.

| Concentration | Correlation coefficient r |
|---|---|
| Fig. 1(a) 0.05% | 0.9085 |
| Fig. 1(b) 0.07% | 0.9152 |
| Fig. 1(c) 0.09% | 0.9198 |
| Fig. 2(d) 0.11% | 0.9263 |
| Fig. 2(e) 0.13% | 0.9304 |
| Fig. 2(f) 0.16% | 0.9295 |
| Fig. 3(g) 0.18% | 0.9296 |
| Fig. 3(h) 0.20% | 0.9252 |
| Fig. 3(i) 0.30% | 0.9240 |

As seen from Figs. 1(c), 2(d) to 2(f), and 3(g) to 3(i), and the above correlation coefficients, at a concentration of POE monostyrenated phenyl ether of 0.05% to 0.30% in Figs. 1(c), 2(d) to 2(f), and 3(g) to 3(i), all of the correlation coefficients are r > 0.90 and sdLDL-C can be more accurately quantified.

Among these, at a concentration of POE monostyrenated phenyl ether of 0.11% to 0.30%, r > 0.92 and sdLDL-C can be more accurately quantified.

This application claims priority based on Japanese Application No. 2021-070615 filed on April 19, 2021.

## Claims

1. A reagent composition that is used as a first reagent composition for a method of quantifying small dense LDL cholesterol (sdLDL-C) in a sample, the method comprising:
causing the first reagent composition to act on the sample; and
after causing the first reagent composition to act on the sample, applying a second reagent composition for quantifying the sdLDL-C to quantify cholesterol in a remaining lipoprotein,
wherein the first reagent composition has one or two or more activities selected from the group consisting of cholesterol esterase activity, cholesterol oxidase activity, and sphingomyelinase activity, and contains polyoxyethylene monostyrenated phenyl ether.

2. The reagent composition according to Claim 1, wherein a degree of polymerization n of polyoxyethylene in the polyoxyethylene monostyrenated phenyl ether is 5 or more and 80 or less.

3. The reagent composition according to Claim 1 or 2, wherein a content of the polyoxyethylene monostyrenated phenyl ether in the reagent composition is 0.05% (w/v) or more and 0.6% (w/v) or less with respect to the entire reagent composition.

4. The reagent composition according to any one of Claims 1 to 3, wherein the first reagent composition further comprises at least one activity selected from the group consisting of peroxidase activity and catalase activity.

5. The reagent composition according to any one of Claims 1 to 4,
wherein the first reagent composition comprises any one of a hydrogen donor and/or a coupler.

6. A kit used for quantifying the sdLDL-C in the sample, comprising:
a first reagent composition according to any one of Claims 1 to 5; and
a second reagent composition for quantifying the sdLDL-C.

7. The kit according to Claim 6,
wherein the second reagent composition has peroxidase activity.

8. The kit according to Claim 6 or 7,
wherein
(i) the first reagent composition comprises a hydrogen donor and does not comprise a coupler, and the second reagent composition does not comprise the hydrogen donor and comprises the coupler; or
(ii) the first reagent composition does not comprise a hydrogen donor and comprises a coupler, and
the second reagent composition comprises the hydrogen donor and does not comprise the coupler.

## Patentansprüche

1. Reagenzzusammensetzung, die als eine erste Reagenzzusammensetzung für ein Verfahren zum Quantifizieren von kleinem, dichtem LDL-Cholesterin (sdLDL-C) in einer Probe verwendet wird, wobei das Verfahren umfasst:
Bewirken, dass die erste Reagenzzusammensetzung auf die Probe einwirkt; und
nach Bewirken, dass die erste Reagenzzusammensetzung auf die Probe einwirkt, Anwenden einer zweiten Reagenzzusammensetzung zum Quantifizieren des sdLDL-C, um Cholesterin in einem verbleibenden Lipoprotein zu quantifizieren,
wobei die erste Reagenzzusammensetzung eine oder zwei oder mehr Aktivitäten, ausgewählt aus der Gruppe, bestehend aus Cholesterinesterase-Aktivität, Cholesterinoxidase-Aktivität und Sphingomyelinase-Aktivität, aufweist und Polyoxyethylen-monostyrolisiertes Phenyl-Ether enthält.

2. Reagenzzusammensetzung gemäß Anspruch 1, wobei der Polymerisationsgrad n von Polyoxyethylen in dem Polyoxyethylen-monostyrolisiertes Phenyl-Ether 5 oder mehr und 80 oder weniger beträgt.

3. Reagenzzusammensetzung gemäß Anspruch 1 oder 2, wobei der Gehalt des Polyoxyethylen-monostyrolisiertes Phenyl-Ethers in der Reagenzzusammensetzung 0,05% (w/v) oder mehr und 0,6% (w/v) oder weniger, bezogen auf die gesamte Reagenzzusammensetzung, beträgt.

4. Reagenzzusammensetzung gemäß einem jeglichen der Ansprüche 1 bis 3, wobei die erste Reagenzzusammensetzung ferner mindestens eine Aktivität, ausgewählt aus der Gruppe, bestehend aus Peroxidase-Aktivität und Katalase-Aktivität, umfasst.

5. Reagenzzusammensetzung gemäß einem jeglichen der Ansprüche 1 bis 4,
wobei die erste Reagenzzusammensetzung ein jegliches aus einem Wasserstoffdonor und/oder einem Kupplungsmittel umfasst.

6. Kit, der zum Quantifizieren des sdLDL-C in der Probe verwendet wird, umfassend:
eine erste Reagenzzusammensetzung gemäß einem jeglichen der Ansprüche 1 bis 5; und
eine zweite Reagenzzusammensetzung zum Quantifizieren des sdLDL-C.

7. Kit gemäß Anspruch 6,
wobei die zweite Reagenzzusammensetzung Peroxidase-Aktivität aufweist.

8. Kit gemäß Anspruch 6 oder 7,
wobei
(i) die erste Reagenzzusammensetzung einen Wasserstoffdonor umfasst und ein Kupplungsmittel nicht umfasst, und
die zweite Reagenzzusammensetzung den Wasserstoffdonor nicht umfasst und das Kupplungsmittel umfasst; oder
(ii) die erste Reagenzzusammensetzung einen Wasserstoffdonor nicht umfasst und ein Kupplungsmittel umfasst, und
die zweite Reagenzzusammensetzung den Wasserstoffdonor umfasst und das Kupplungsmittel nicht umfasst.

## Revendications

1. Composition de réactif qui est utilisée comme première composition de réactif pour un procédé de quantification de cholestérol LDL dense de petite taille (sdLDL-C) dans un échantillon, le procédé comprenant :
le fait d'amener la première composition de réactif à agir sur l'échantillon ; et
après avoir amené la première composition de réactif à agir sur l'échantillon, l'application d'une seconde composition de réactif pour quantifier le sdLDL-C afin de quantifier le cholestérol dans une lipoprotéine restante,
dans laquelle la première composition de réactif possède une ou deux activités ou plus choisies dans le groupe constitué par l'activité cholestérol estérase, l'activité cholestérol oxydase et l'activité sphingomyélinase, et contient un éther phénylique monostyréné de polyoxyéthylène.

2. Composition de réactif selon la revendication 1, dans laquelle un degré de polymérisation n de polyoxyéthylène dans l'éther phénylique monostyréné de polyoxyéthylène est supérieur ou égal à 5 et inférieur ou égal à 80.

3. Composition de réactif selon l'une des revendications 1 ou 2, dans laquelle une teneur en éther phénylique monostyréné de polyoxyéthylène dans la composition de réactif est de 0,05 % (p/v) ou plus et de 0,6 % (p/v) ou moins par rapport à la composition de réactif totale.

4. Composition de réactif selon l'une quelconque des revendications 1 à 3, dans laquelle la première composition de réactif comprend en outre au moins une activité choisie dans le groupe constitué par l'activité peroxydase et l'activité catalase.

5. Composition de réactif selon l'une quelconque des revendications 1 à 4, dans laquelle la première composition de réactif comprend l'un quelconque d'un donneur d'hydrogène et/ou d'un coupleur.

6. Kit utilisé pour quantifier le sdLDL-C dans l'échantillon, comprenant :
une première composition de réactif selon l'une quelconque des revendications 1 à 5 ; et
une seconde composition de réactif pour quantifier le sdLDL-C.

7. Kit selon la revendication 6, dans lequel la seconde composition de réactif présente une activité peroxydase.

8. Kit selon l'une des revendications 6 ou 7, dans lequel
(i) la première composition de réactif comprend un donneur d'hydrogène et ne comprend pas de coupleur, et la seconde composition de réactif ne comprend pas le donneur d'hydrogène et comprend le coupleur ;
ou
(ii) la première composition de réactif ne comprend pas de donneur d'hydrogène et comprend un coupleur, et la seconde composition de réactif comprend le donneur d'hydrogène et ne comprend pas le coupleur.
